# EUROPEAN PATENT APPLICATION

(11) **EP 2 551 283 A1**
(43) Date of publication of application: **30.01.2013**
(21) Application number: 11759357.4
(22) Date of filing: 22.03.2011
(51) Int. Cl.: C08B 37/00, A61K 31/715, A61K 35/74, A61P 17/00, A61P 37/08

(54) **ANTI-ALLERGIC AGENT**

(30) Priority: 24.03.2010 JP 2010067639
(71) Applicant: Morishita Jintan Co., Ltd., Osaka-shi Osaka 540-8566 (JP)
(72) Inventor: KOHNO, Mamiko, Osaka-shi Osaka 540-8566 (JP); KITAMURA, Shinichi, Sakai-shi Osaka 599-8531 (JP); SHOJO, Aiko, Sakai-shi Osaka 599-8531 (JP)
(74) Representative: Schlich, George William
(86) International application number: PCT/JP2011/056733
(87) International publication number: WO 2011/118552

(57) **Abstract**

According to the present invention, an anti-allergic agent is provided which includes a polysaccharide comprising galactose, glucose and rhamnose as constituents, or includes a microorganism belonging to the genus *Bifidobacterium* and extracellularly producing the polysaccharide. The anti-allergic agent of the present invention can be used in oral compositions and compositions for external application, and is suitable for use in products such as food products, pharmaceutical products, and cosmetics.

## Description

### Technical Field

The present invention relates to an anti-allergic agent.

### Background Art

For a microorganism belonging to the genus *Bifidobacterium* (hereinafter also referred to as "bifidobacterium"), one of the dominant bacteria in the intestine, many reports have been made on the effects of such a microorganism on intestinal regulations and immunoregulations.

Some bifidobacteria have been reported as producing polysaccharides extracellularly. Many of the reports pertain to the characteristic constituents and structures of the polysaccharides. Polysaccharides produced by bifidobacteria have been reported on their immunostimulating function (Patent Documents 1 and 2).

There are some reports on anti-allergic effects of polysaccharides produced by microorganisms other than bifidobacteria. Patent Document 3 describes an anti-inflammatory drug which contains as an active ingredient a polysaccharide obtained from the culture broth of *Lactobacillus* bacterium, and Patent Document 4 describes an immunomodulator which contains a polysaccharide produced by *Lactobacillus fermentum* as an active ingredient that has an anti-inflammatory effect, an anti-allergic effect and a tumor proliferation inhibiting effect. Patent Document 5 describes an allergy-suppressive composition which contains a levan polysaccharide produced by *Bacillus subtilis*var*. natto* as an active ingredient.

*Bacillus subtilis* var. *natto* is not a microorganism inhabiting the intestine as an indigenous bacterium. In the healthy intestine, bifidobacteria are overwhelmingly dominant over lactic bacteria. There has not yet been any report on an anti-allergic effect of bifidobacteria which produce an extracellular polysaccharide, among bifidobacteria which are dominant bacteria in the intestine.

### Prior Art Documents

### Patent Documents

Patent Document 1: WO 07/007562
Patent Document 2: Japanese Laid-Open Patent Publication No. 58-203913
Patent Document 3: Japanese Laid-Open Patent Publication No. 7-70209
Patent Document 4: Japanese Laid-Open Patent Publication No. 2008-245576
Patent Document 5: Japanese Laid-Open Patent Publication No. 2006-1922

### Summary of Invention

### Problems to be Solved by the Invention

An object of the present invention is to provide an anti-allergic agent with no risk of any side effect.

### Means for Solving the Problems

The present invention provides an anti-allergic agent including a polysaccharide including galactose, glucose and rhamnose as constituents.

In an embodiment, the polysaccharide includes galactose, glucose and rhamnose in a molar ratio of 3-5 : 1-3 :1.

In an embodiment, the polysaccharide includes the following structure:

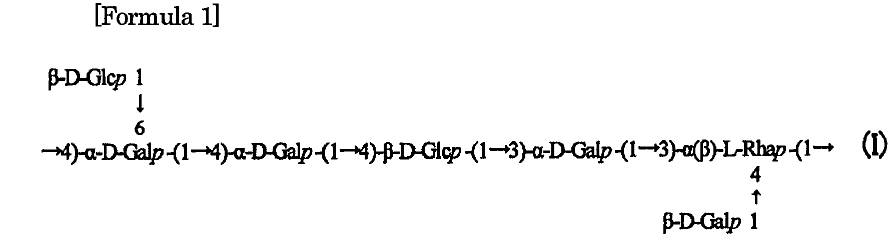

In an embodiment, the polysaccharide is obtained from a microorganism belonging to the genus *Bifidobacterium.*

In a further embodiment, the microorganism belonging to the genus *Bifidobacterium* is *Bifidobacterium longum.*

In a still further embodiment, the *Bifidobacterium longum* is *Bifidobacterium longum* strain JBL05 (NITE BP-82).

The present invention also provides an anti-allergic agent including a microorganism, the microorganism belonging to a genus *Bifidobacterium* and producing an extracellular polysaccharide as mentioned above.

In an embodiment, the bifidobacterium which produces an extracellular polysaccharide as mentioned above is *Bifidobacterium longum* strain JBL05 (NITE BP-82).

The present invention also provides an oral composition including the anti-allergic agent. This anti-allergic agent is an anti-allergic agent including the bifidobacterially produced polysaccharide mentioned above or an anti-allergic agent including the exopolysaccharide-producing bifidobacterium mentioned above.

The present invention also provides a composition for external application including the anti-allergic agent. This anti-allergic agent is an anti-allergic agent including the bifidobacterially produced polysaccharide mentioned above.

In an embodiment, the oral composition or composition for external application is for suppressing atopic dermatitis or contact dermatitis.

### Effects of Invention

The present invention provides an anti-allergic agent exhibiting a beneficial allergy-suppressing effect with no risk of any side effect.

### Brief Description of Drawings

[Fig. 1] Fig. 1 show graphs indicating production of interferon-γ (IFN-γ) (A), production of interleukin-4 (IL-4) (B) and the ratio between IFN-γ and IL-4 produced (C) in response to *in vitro* stimulation with the bifidobacterially produced polysaccharide to splenocytes.
[Fig. 2] Fig. 2 is a graph showing a change over time of average auricular thickness in mice receiving oral administration of the bifidobacterially produced polysaccharide after the beginning of sensitization.
[Fig. 3] Fig. 3 shows photomicrographs of H&E-stained section from the auricles of treated mice including mice receiving oral administration of the bifidobacterially produced polysaccharide on Day 20 after the beginning of sensitization.
[Fig. 4] Fig. 4 is a graph showing a change over time of average auricular thickness in mice receiving orally administrated exopolysaccharide-producing bifidobacterium after the beginning of sensitization.
[Fig. 5] Fig. 5 shows photomicrographs of H&E-stained sections from the auricles of treated mice including mice receiving oral administration of the exopolysaccharide-producing bifidobacterium on Day 20 after the beginning of sensitization.
[Fig. 6] Fig. 6 is a graph showing a change over time of average auricular thickness in mice receiving applications of the bifidobacterially produced polysaccharide after the beginning of sensitization.

### Mode for Carrying Out the Invention

### (Bifidobacterially produced polysaccharide and exopolysaccharide-producing bifidobacterium)

In the present invention, a polysaccharide containing galactose, glucose and rhamnose as constituents is used. The polysaccharide may contain galactose : glucose : rhamnose in a molar ratio of 3-5 : 1-3 : 1. The polysaccharide in which the molar ratio of galactose : glucose : rhamnose is e.g. 4 : 2 : 1 may have a repeated structure represented by the following formula (I).

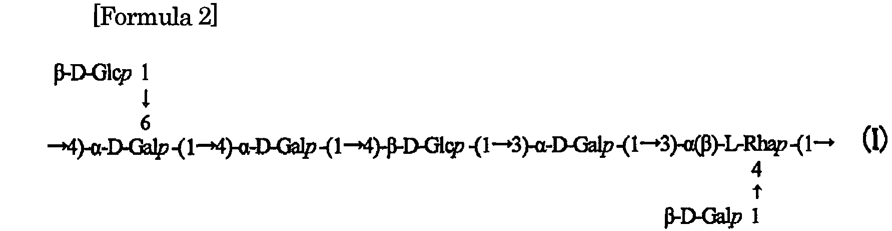

In formula (I), Galp is galactopyranose, Glcp is glucopyranose and Rhap is rhamnopyranose, meaning that galactose, glucose and rhamnose have a pyranose structure. The above-mentioned polysaccharide may further contain pyruvic acid, and the pyruvic acid may be contained in the polysaccharide at a rate of up to 10 % by weight. Identification of the constituents of the polysaccharide, determination of the amounts or rates of the constituents in the polysaccharide and structural analysis of the polysaccharide will be described in detail below. The polysaccharide having the structure may be produced by a microorganism belonging to the genus *Bifidobacterium,* or in particular *Bifidobacterium longum,* e.g. *Bifidohacterium longum* strain JBL05 (IVITE BP-82) which is isolated from the human intestine. Therefore, this polysaccharide is, for convenience, herein also referred to as the "bifidobacterially produced polysaccharide", but by this phrase is not meant to limit microorganisms that produce the polysaccharide.

In the present invention, a microorganism belonging to the genus *Bifidobacterium* and producing an extracellular polysaccharide as mentioned above is also used. The phrase "producing an extracellular polysaccharide" means that a microorganism produces a capsule-like polysaccharide around itself. This microorganism is, for convenience, herein also referred to as the "exopolysaccharide-producing bifidobacterium".

In the present invention, *Bifidobacterium longum* strain JBL05 (NITE BP-82) is exemplified as a microorganism used to prepare the bifidobacterially produced polysaccharide or as the exopolysaccharide-producing bifidobacterium. The bacteriological properties of *Bifidobacterium longum* strain JBL05 (NITE BP-82) are as shown in Table 1 below.

[Table 1]

| A. Morphological properties | | |
|---|---|---|
| (1) | Shape | 0.6 to 0.8 X 1.0 to 4.0 µm, clavate, Y-shaped, and curved Gram-positive bacillus |
| (2) | Motility | - |
| (3) | Spore | - |

| B. Culture properties *¹ | | |
|---|---|---|
| (1) | Shape | Round circle, smooth both on the surface and the circumference |
| (2) | Size | Diameter 0.5 to 2.5 mm |
| (3) | Protrusion | Protrude in the shape of a hemisphere |
| (4) | Color | Yellowish-brown or milky white |
| (5) | Characteristics | Slightly viscous |
| | | *1 Properties of a colony obtained by application to a BL agar medium (NISSUI PHARMACEUTICAL CO., LTD.), followed by cultivation for 48 hours at 37°C under anaerobic conditions in a closed container containing AnaeroPack (MITSUBISHI GAS CHEMICAL COMPANY, INC.) |

| C. Physiological properties | | |
|---|---|---|
| (1) | Nitrate reduction | - |
| (2) | Indole production | - |
| (3) | Catalase | - |
| (4) | Optimum growth temperature | 37°C |
| (5) | Optimum pH | pH 6.5 to 7.0 |
| (6) | Degree of anaerobicity | Obligately-anaerobic |
| (7) | Gas production | - |
| (8) | Sugar assimilation | |
| | Arabinose | + |
| | Xylose | + |
| | Ribose | + |
| | Glucose | + |
| | Galactose | + |
| | Mannose | + |
| | Fructose | + |
| | Saccharose | + |
| | Maltose | + |
| | Cellobiose | - |
| | Lactose | + |
| | Trehalose | - |
| | Melibiose | + |
| | Raffinose | + |
| | Melezitose | + |
| | Starch | ± |
| | Glycerin | - |
| | Mannitol | - |
| | Sorbitol | - |
| | Inositol | - |
| | (+: Positive, -: Negative, ±: Slightly positive) | |

Based on the taxonomic characteristics of the phenotype, strain JBL05 was identified as *Bifidobacterium longum* according to Bergey's Manual of Systematic Bacteriology, Vol. 2 (1984), and was deposited with Incorporated Administrative Agency, National Institute of Technology and Evaluation, Patent Microorganisms Depositary (NPMD) (address: 2-5-8 Kazusakamatari, Kisarazu-shi, Chiba-ken 292-0818, Japan) on March 3, 2005 (originally deposited date), and accepted as NITE BP-82.

To prepare the polysaccharide, *Bifidobacterium longum* strain JBL05 (NITE BP-82) can be cultured in an appropriate culture medium. The procedure of culturing will be illustrated below, but not limited thereto.

An example of the culture medium is a culture medium that contains a carbon source and a nitrogen source that can be used by microorganisms belonging to the genus *Bifidobacterium,* and optionally contains cysteine hydrochloride, sodium ascorbate, and a trace amount of inorganic salts. In particular, a culture medium containing skim milk or a milk constituent is preferable to produce a large amount of polysaccharide, and a culture medium that contains enzymatically-degraded skim milk, Cultivator (a fish extract manufactured by Yaizu Suisankagaku Industry Co., Ltd.), lactose and sodium ascorbate can be preferably used, wherein the enzymatically-degraded skim milk is obtained by degrading skim milk with an enzyme such as protease.

When *Bifidobacterium longum* strain JBL05 (NITE BP-82) is anaerobically cultured in this medium with stirring or standing at culture temperatures from 20 to 45 °C for 12 to 60 hours, preferably 15 to 50 hours while adjusted to pH 4 to 7, preferably 5 to 6, a viscous substance (polysaccharide) can be produced in a culture broth.

The polysaccharide can be collected from the resultant culture broth using methods commonly used by those skilled in the art to collect a substance of interest from a culture broth, including heating, enzyme treatment, centrifugation, filtration, membrane processing and filtering out. For example, the culture broth which contains a viscous substance (polysaccharide) and microorganism cells is centrifuged to remove the cells. When the viscosity of the culture broth is high, the culture broth may be diluted with water and then centrifuged to remove the cells, for example. Subsequently, the resultant supernatant is treated with an appropriate organic solvent to precipitate proteins, and the precipitate is removed by centrifugation or the like. Further, the supernatant is treated with an organic solvent to precipitate the polysaccharide, and the polysaccharide is collected by centrifugation or the like. More specifically, to the supernatant, from which the cells have been removed, ethanol is added to a final concentration of 20 % by volume, and then centrifuged to remove a protein-containing precipitate, and to the resultant supernatant, ethanol is added to a final concentration of 50 % by volume, and then centrifuged, and the precipitate is collected, thereby obtaining a crude polysaccharide.

Alternatively, it is also possible to employ methods for collecting a polysaccharide with the combination of an organic solvent and a cation(s). For example, methods for collection with monovalent cation(s) and an organic solvent, collection with bivalent cation(s) and an organic solvent (Japanese Laid-Open Patent Publication No. 58-5301), and collection with trivalent cation(s) and an organic solvent (Japanese Laid-Open Patent Publication No. 59-196099), and the like method can be employed. Such a cation(s) is preferably used to improve the rate of collection of the polysaccharide. Examples of monovalent cation(s) include sodium and potassium, ions and the like; examples of bivalent cation(s) include magnesium and calcium, ions and the like; and examples of trivalent cation(s) include aluminum ions and the like. Such a cation can be added to a viscous solution, which contains the polysaccharide, together with an organic solvent such as ethanol to collect the polysaccharide in a larger amount. To improve the rate of collection of the polysaccharides, bivalent or trivalent cation(s) can be used rather than monovalent cation(s).

The polysaccharide can be purified from the crude polysaccharide using methods commonly used by those skilled in the art, for example, fractionation with an ion exchange resin or fractionation by gel filtration, alone or in combination. There is no specific limitation on the process with an ion exchange resin. For example, the polysaccharide is adsorbed to an anion exchange resin (for example, product name: DEAE Sephadex A-50 (Pharmacia Corporation), or the like), and then eluted with a gradient of sodium chloride. Examples of the process by gel filtration include those with a product named TOYOPEARL HW65S (Tosoh Corporation), and the like.

The structure of the polysaccharide can be determined in a manner set forth below. The purified polysaccharide is acid-hydrolyzed with an acid such as formic acid, dilute hydrochloric acid or trifluoroacetic acid, and the hydrolysate is then subjected to HPLC to determine saccharides (monosaccharides) constituting the polysaccharide. Then, the acid-hydrolysate is conventionally acetylated and then subjected to gas chromatography (GC) to determine the composition of the constituent saccharides (the ratio between the constituent saccharides). Furthermore, the purified polysaccharide is conventionally methylated and then acid-hydrolyzed, and the hydrolysate is reduced and then acetylated, and the resultant is subjected to GC-MS (gas chromatography / mass spectroscopy) to determine the type of the glycoside linkage for constituents of the polysaccharide. Moreover, the polysaccharide is analyzed by NMR to reveal the glycosidic linkage of monosaccharides to each other. The pyruvic acid bonded to the polysaccharide can be qualitatively and quantitatively determined by measuring the reduction of pyruvic acid into lactic acid with lactate dehydrogenase in the presence of NADH.

*Bifidobacterium longum* strain JBL05 (NITE BP-82) can produce a polysaccharide having the following characteristics:
(1) the polysaccharide contains galactose, glucose and rhamnose as constituents;
(2) the polysaccharide has a molar ratio of galactose: glucose: rhamnose of 3-5: 1-3: 1;
(3) the polysaccharide may contain pyruvic acid in a rate of up to 10 % by weight;
(4) the polysaccharide has a molecular weight in the range of about 50,000 to 10,000,000, preferably about 200,000 to 2,500,000 (as determined by gel permeation chromatography / multiangle laser light scattering detection (GPC-MALLS)); and
(5) the polysaccharide possibly has a repeated structure as represented by the following formula (I) wherein the polysaccharide has a molar ratio of galactose : glucose : rhamnose of e.g. 4 : 2 : 1.

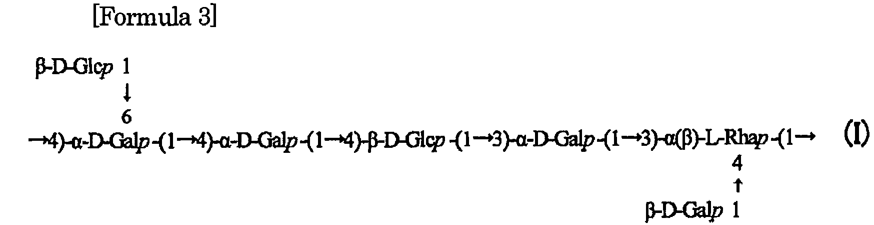

The polysaccharide having the above-mentioned characteristics may be contained in the purified polysaccharide fraction obtained following the procedure described in Example 1 below.

The molecular weight of the polysaccharide varies depending on the culture conditions and collection / purification conditions. Also, the molecular weight can be adjusted by selecting suitable culture conditions and the like.

The polysaccharide having the structure may be produced by *Bifidobacterium longum* strain JBL05 (NITE BP-82) isolated from the human intestine although other microorganisms may be used to prepare the polysaccharide. Such microorganisms may be identified by, for example, isolating intestinal bacteria and analyzing polysaccharides derived from strains determined to produce capsule-like polysaccharides around the bacteria, and collection and purification of the polysaccharide may be performed according to the above.

*Bifidobacterium longum* strain JBL05 (NITE BP-82) is exemplified as an exopolysaccharide-producing bifidobacterium although other bifidobacteria extracellularly producing the bifidobacterially produced polysaccharide may be also used in the present invention.

The exopolysaccharide-producing bifidobacterium for use in the present invention may be those that contain bacterial cells obtained from the culture broth after the bifidobacterium is cultured for preparation of the polysaccharide. As the exopolysaccharide-producing bifidobacterium, the bifidobacterium culture broth after culturing may be used directly, or through centrifugation, membrane process, filtration or the like to give a cell-containing culture broth. Such a culture broth also may be used in a concentrated, dried or crushed form or the like. Examples of the drying include vacuum drying, spray drying, lyophilization, drum drying, and the like. Together with bacterial cells, dispersants, tritulations and the like may be contained in the exopolysaccharide-producing bifidobacterium. The bifidobacteria may be prepared as viable bacteria according to the commonly used procedure. A culture broth containing the bacterial cells directly, or in a concentrated or dried form or the like may be subjected to sterilization such as heat sterilization and irradiation, optionally followed by homogenization, to obtain killed bacteria which can be also used in the present invention. The procedures used to prepare such bacterial cells are in accordance with the procedure commonly used by those skilled in the art.

### (Anti-allergic agent)

The bifidobacterially produced polysaccharide and the exopolysaccharide-producing bifidobacterium exhibit an anti-allergic effect. Anti-allergic effects include, but are not limited to, Th1/Th2 balance-improving effect, Th17 modulating effect, inflammatory suppressing effect, IgE production-inhibiting effect, chemical mediator (such as histamine) release-inhibiting effect and lymphocyte activating effect. Therefore, according to the present invention, the bifidobacterially produced polysaccharide or the exopolysaccharide-producing bifidobacterium can be used as an anti-allergic agent, and an anti-allergic agent containing the bifidobacterially produced polysaccharide or the exopolysaccharide-producing bifidobacterium is provided.

The bifidobacterially produced polysaccharide or the exopolysaccharide-producing bifidobacterium may be contained as an anti-allergic agent in any composition. Effects of the anti-allergic agent include, but are not limited to, suppression of type I allergy, type IV allergy, atopic dermatitis, contact dermatitis, allergic dermatitis, pollinosis, allergic rhinitis, allergic conjunctivitis and bronchial asthma. Such compositions are useful for prevention or treatment of allergic symptoms. Preferably, they can suppress atopic dermatitis or contact dermatitis.

Such compositions can be used for food products, pharmaceutical products and cosmetics. The compositions may be prepared and used particularly as, for example, but not limited to, powders, granules, tablets, capsules, pastes, creams, gels and liquids. The compositions may be used after being mixed with inorganic or organic substances such as excipients, binders, disintegrants, lubricants, correctives, solubilizers, suspending agents and coating agents as appropriate. The compositions may be produced using methods well known to those skilled in the art for the manufacture of food products, pharmaceutical products and cosmetics.

The formulated amount of the bifidobacterially produced polysaccharide may be suitably determined depending on the purpose, intended use, form, dosage form, symptom, body weight and the like, and, for oral intake or administration, the compositions are preferably prepared such that 1 mg to 10 g, more preferably 10 mg to 2 g of the bifidobacterially produced polysaccharide per day can be taken. For administration by application to the skin or mucosa, the formulated amount of the bifidobacterially produced polysaccharide is preferably 0.001 to 10 % by weight, more preferably 0.01 to 1 % by weight of the total weight of the product.

The formulated amount of the exopolysaccharide-producing bifidobacterium may be also suitably determined depending on the purpose, intended use, form, dosage form, symptom, body weight and the like, and the bifidobacterium is contained in the product such that preferably 10⁶ to 10¹², more preferably 10⁸ to 10¹¹ cells per day can be taken.

The compositions containing the bifidobacterially produced polysaccharide or the exopolysaccharide-producing bifidobacterium may be used as oral compositions. For example, the oral compositions may be used as food compositions or pharmaceutical compositions because of their anti-allergic effect via oral ingestion, and can be added to pharmaceutical products for oral administration, fluid diet, invalid diet products, infant food products, foods with nutrient function claims, foods for specified health uses and the like. The compositions containing the bifidobacterially produced polysaccharide or the exopolysaccharide-producing bifidobacterium may be formulated into ordinary food and drink products, for example, including, but not limited to, various beverages such as milk, soft drinks and jelly drinks, various confectioneries such as candies, gummi candies, chocolates, biscuits and crackers and various food products such as rice, bread, udon noodles and dressings. The compositions containing viable cells of the exopolysaccharide-producing bifidobacterium may be used for the fermentation of milk etc. into yogurt and fermented milk to be taken.

The compositions containing the bifidobacterially produced polysaccharide may be used as compositions for external application. For example, the compositions for external application may be used as cosmetic compositions or pharmaceutical compositions because of their anti-allergic effect via application or administration to the skin or mucosa. The cosmetics into which the compositions may be formulated include, but are not limited to, for example, facial washes, skin lotions, serum, milky lotions, sprays, mask sheets, creams, ointments, bath additives and the like. The cosmetic compositions may also include ones produced and sold as quasi drugs. The pharmaceutical products into which the compositions may be formulated include, but are not limited to, compositions for administration to the nasal mucosa (e.g. nasal drops and sprays), compositions for administration to the ocular mucosa (e.g. eye drops and eye washes), compositions for administration to the throat mucosa (e.g. gargle medicines, mouthwashes, sprays and troches) and the like. Such pharmaceutical products also include hygiene products such as patches, surgical dressings and adhesive plasters.

The present invention shall be described in more detail in examples below, but the present invention is not limited thereto.

### Examples

### (Example 1: Culturing of exopolysaccharide-producing bifidobacterium and preparation of bifidobacterially produced polysaccharide)

Culturing of *Bifidobacterium longum* strain JBL05 (NITE BP-82) and purification of the polysaccharide produced by the microorganism were performed as described in Patent Document 1, which are described in detail below.

To a 9 % by weight solution of skim milk, pancreatin (Amano Enzyme Inc.) and silicon were added to final concentrations of 0.36 % by weight and 0.01 % by weight, respectively, and the resultant was adjusted to pH 8 with 10N NaOH, and allowed to react at 55°C for four hours, thereby obtaining an enzymatically-degraded skim milk. To the enzymatically-degraded skim milk, Cultivator (a fish extract manufactured by Yaizu Suisankagaku Industry Co., Ltd.), lactose, and sodium ascorbate were added to final concentrations of 3.0 % by weight, 2.5 % by weight, and 0.2 % by weight, respectively, and then sterilized in an autoclave at 121°C for 15 minutes. The resultant was used as a liquid culture medium.

*Bifidobacterium longum* strain JBL05 (NITE BP-82) was pre-cultured in the liquid medium prepared as mentioned above, and then inoculated in 5 L of the same liquid medium to a concentration of 1 % (v/v), and anaerobically cultured with standing at 37°C for 40 hours to produce a viscous substance. The culture broth was centrifuged to remove bacterial cells. Subsequently, ethanol was added to the supernatant to a final concentration of 20 %, and the resultant was allowed to stand at 4°C overnight, and was then centrifuged to remove a protein-containing precipitate. Further, ethanol was added to the supernatant to a final concentration of 50 %, and the resultant was allowed to stand at 4°C overnight and was then centrifuged, and the precipitate was collected, thereby obtaining a crude polysaccharide fraction. The fraction was lyophilized and stored.

The crude polysaccharide fraction was further fractionated with a column filled with DEAE Sephadex A-50, and eluted with 0.07M to 0.5M of NaCl. The resultant fraction was lyophilized to give a purified polysaccharide fraction.

### (Example 2: Structural analysis of bifidobacterially produced polysaccharide)

The purified polysaccharide fraction prepared in Example 1 was subjected to gel filtration with a column filled with TOYOPEARL HW65S, and then examined for the molecular weight by GPC-MALLS, and the polysaccharide was found to have a molecular weight of approximately 540,000.

Next, the gel-filtrated fraction (polysaccharide) was hydrolyzed with formic acid, and the hydrolysate was dried under reduced pressure. Then, the resultant was hydrolyzed with trifluoroacetic acid to give a hydrolysate product. The hydrolysate product was subjected to HPLC with an ION-300 column (Tokyo Chemical Industry Co., Ltd.). Accordingly, the polysaccharide was found to be constituted by galactose, glucose and rhamnose.

The hydrolysate product was conventionally reduced with sodium borohydride, and was then acetylated with acetic anhydride and pyridine. The resultant was subjected to GC with an R-225 column (J&W Scientific). Accordingly, the polysaccharide was found to be constituted at the molar ratio of galactose, glucose and rhamnose of 4 : 2 :1.

When the hydrolysate product was treated with lactate dehydrogenase in the presence of NADH, lactic acid was generated, thereby identifying the presence of pyruvic acid in the polysaccharide fraction, and also the content of pyruvic acid in the polysaccharide of 5 % by weight.

In order to clarify the type of the glycoside linkage of constituents of the polysaccharide, methylation analysis of the polysaccharide was performed. The gel-filtrated fraction (polysaccharide) was conventionally methylated and then hydrolyzed with formic acid, and the hydrolysate was reduced and then acetylated. The resultant was subjected to GC-MS. Accordingly, methylated saccharides were obtained as shown below: 1,5-di-*O-*acetyl-2,3,4,6-tetra-*O-*methyl-glucitol, 1,5-di-*O*-acetyl-2,3,4,6-tetra-*O*-methyl-galactitol, 1,3,4,5-tetra-*O*-acetyl-2,6-di-*O*-methyl-rhamnitol, 1,3,5-tri-*O*-acetyl-2,4,6-tri-*O*-methyl-galactitol, 1,4,5-tri-*O*-acetyl-2,3,6-tri-*O*-methyl-glucitol, 1,4,5-tri-*O*-acetyl-2,3,6-tri-*O*-methyl-galactitol, and 1,4,5,6-tetra-*O*-acetyl-2,3-di-*O*-methyl-galactitol.

Furthermore, the glycosidic linkage of the constituents was examined by NMR. It was found from these data that the polysaccharide has the following Structure I (repetitive structure).

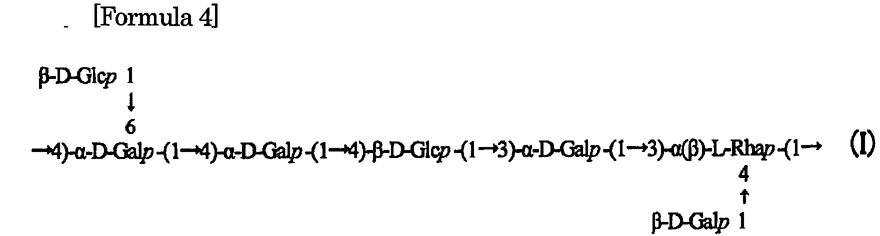

### (Example 3: Th1/Th2 balance-improving effect of bifidobacterially produced polysaccharide)

Splenocytes prepared from C3H/HeJ mice (8 weeks old, male, CLEA Japan, Inc.) were cultured at 37°C for 72 hours under 5% CO₂ in RPMI-1640 medium containing fetal bovine serum and antibiotics (antibiotics-antifungal mixture solution, Nacalai Tesque, Inc.) supplied with the purified polysaccharide fraction prepared in Example 1 which was dissolved in water to a final concentration of 20 µg/ml or 200 µg/ml. The culture supernatant was recovered and the concentrations of interferon-y (IFN-γ), which is a Th1 cytokine, and interleukin-4 (IL-4), which is a Th2 cytokine, were measured with ELISA (BIOSOURCE, Invitrogen). Control culture was performed with the same procedure described above with the exception that the same amount of water was added in place of the purified polysaccharide fraction.

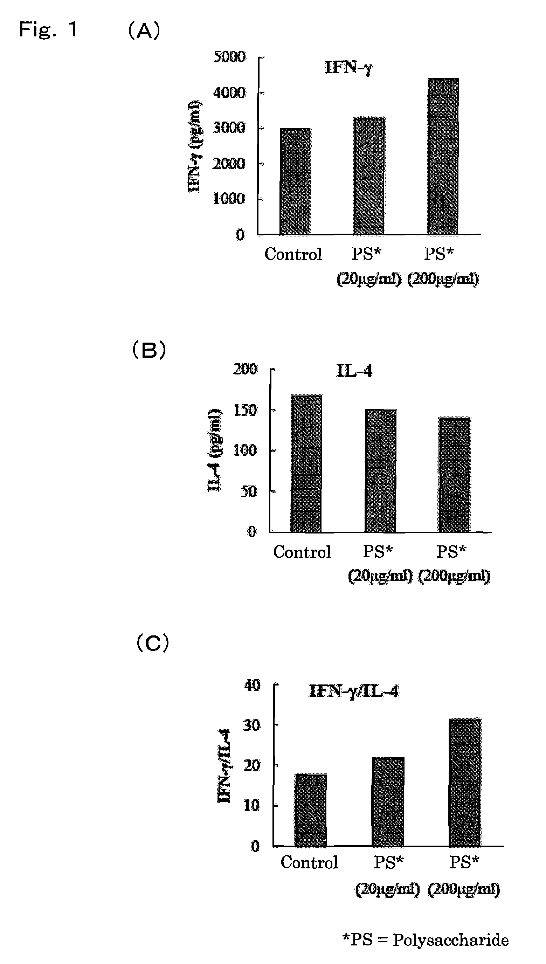

Results are shown in Figs. 1(A) to (C). Fig. 1(A) is a graph showing the amount of IFN-γ produced in response to *in vitro* stimulation with the bifidobacterially produced polysaccharide to splenocytes. The vertical axis represents the amount of IFN-γ (pg/ml) produced and the horizontal axis shows experimental groups. Fig. 1(B) is a graph showing the amount of IL-4 produced in response to *in vitro* stimulation with the bifidobacterially produced polysaccharide to splenocytes. The vertical axis represents the amount of IL-4 produced (pg/ml) and the horizontal axis shows experimental groups. Fig. 1(C) is a graph showing the ratio between IFN-γ and IL-4 produced in response to *in vitro* stimulation with the bifidobacterially produced polysaccharide to splenocytes. The vertical axis represents the ratio of IFN-γ to IL-4 (IFN-γ/IL-4) and the horizontal axis shows experimental groups.

These results demonstrated that addition of the purified polysaccharide fraction (bifidobacterially produced polysaccharide) increased the amount of IFN-γ produced and decreased the amount of IL-4 produced in concentration-dependent manner as compared to the control culture with the same amount of water added in place of the purified polysaccharide fraction.

Therefore, it was shown that addition of the bifidobacterially produced polysaccharide increases Th1 cytokine and thus Th1 becomes predominant in the balance between Th1/Th2, that is, providing an anti-allergic effect.

### (Example 4: Anti-allergic effect of bifidobacterially produced polysaccharide)

The purified polysaccharide fraction prepared in Example 1 was dissolved in phosphate buffer (PBS) and orally administered to five BALB/c mice (8 week-old, male, Kiwa Laboratory Animals Co., Ltd.) with feeding probes every day (20 mg/kg body weight/day). For the control group, PBS was orally administrated alone to five mice every day. For the positive control group, prednisolone (Sigma) was orally administered to five mice every day (3 mg/kg body weight/day). On Day 4 after the beginning of the administration, 10 µl of 0.3 % 2,4,6-trinitro-1-chlorobenzene (TNCB) (Tokyo Chemical Industry Co., Ltd.) dissolved in acetone (Nacalai Tesque, Inc.) was applied (sensitization) to their right auricles and the same amount of acetone was applied alone to their left auricles, which were repeated every other day from Day 4 to Day 19 after the beginning of the TNCB application (sensitization). Oral administration as described above was continued also after the beginning of the sensitization. The thickness of auricle was measured using a caliper for every TNCB application from the first day of the sensitization. The measurements were performed every day by the same examiner under the same conditions, and the average values were calculated (five mice per group). Day 20 after the beginning of the sensitization, auricles were observed histologically. For the histological observation, after the dissection, sections were prepared from auricles fixed with 10% neutral buffered formalin (Wako Pure Chemical Industries, Ltd.), and then stained with hematoxylin-eosin (H&E) (commissioned to Applied Medical Research Laboratory) and observed with a microscope.

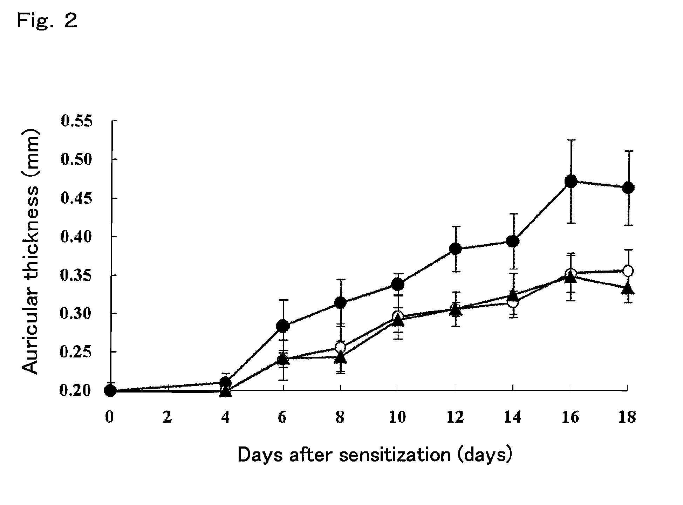

Fig. 2 is a graph displaying a change over time of average auricular thickness in mice receiving oral administration of the bifidobacterially produced polysaccharide after the beginning of the sensitization (five mice per group). The vertical axis represents the auricular thickness (mm), and the horizontal axis represents time after the sensitization (days). Filled circles represent the control (PBS alone) group, open circles represent the positive control (prednisolone) group, filled triangles represent the group receiving administration of the bifidobacterially produced polysaccharide.

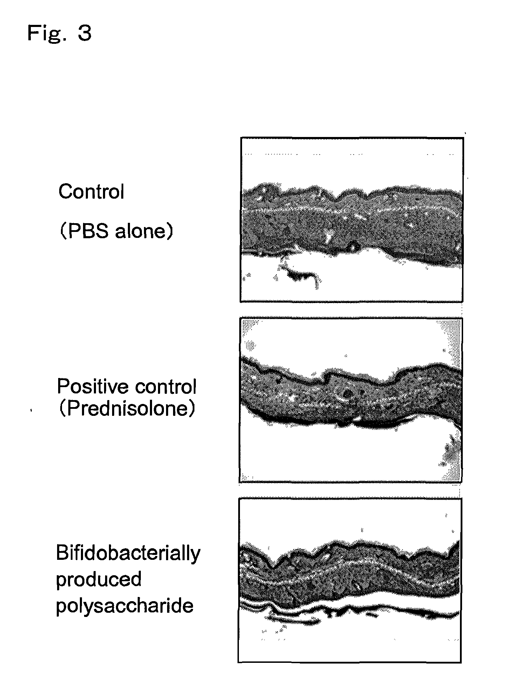

Fig. 3 shows photomicrographs of H&E-stained sections from the auricles of treated mice including mice receiving oral administration of the bifidobacterially produced polysaccharide on Day 20 after the beginning of the sensitization. The photomicrographs are, starting from the top, pictures of the control (PBS alone) group, the positive control (prednisolone) group and the group receiving administration of the bifidobacterially produced polysaccharide.

Oral administration of the purified polysaccharide fraction (bifidobacterially produced polysaccharide) significantly inhibited an increase of the auricular thickness as with the positive control receiving oral administration of prednisolone, as compared to control receiving oral administration of PBS (Fig. 2). The results of histological observation of the auricles also showed that oral administration of the purified polysaccharide fraction (bifidobacterially produced polysaccharide) suppresses inflammation induced in the auricle and inhibits an increase of the auricular thickness as with the positive control receiving oral administration of prednisolone (Fig. 3). Thus, the purified polysaccharide fraction exhibited an anti-allergic effect.

### (Example 5: Anti-allergic effect of exopolysaccharide-producing bifidobacterium)

The effect of *Bifidobacterium longum* strain JBL05 (viable bacterial cells) was examined using the same method as in Example 4.

Cells of *Bifidobacterium longum* JBL05, an exopolysaccharide-producing bifidobacterium, were suspended in PBS and then orally administered to five BALB/c mice (8 week-old, male, Kiwa Laboratory Animals Co., Ltd.) with feeding probes every day (10⁸ of viable bacterial cells/mouse/day). For the control group, PBS is orally administrated alone to five mice every day. For the positive control group, prednisolone (Sigma) was orally administered to five mice every day (3 mg/kg body weight/day). On Day 4 after the beginning of the administration, 10 µl of 0.3 % 2,4,6-trinitro-1-chlorobenzene (TNCB) (Tokyo Chemical Industry Co., Ltd.) dissolved in acetone (Nacalai Tesque, Inc.) was applied to their right auricles (sensitization) and the same amount of acetone was applied alone to their left auricles, which were repeated every other day from Day 4 to Day 19 after the beginning of the TNCB application (sensitization). Oral administration as described above was continued also after the beginning of the sensitization. The thickness of auricle was measured using a caliper for every TNCB application from the first day of the sensitization. The measurements were performed by the same examiner under the same conditions, and the average values were calculated (five mice per group). On Day 20 after the beginning of the sensitization, auricles were observed histologically. For the histological observation, after the dissection, sections were prepared from the auricles fixed with 10% neutral buffered formalin (Wako Pure Chemical Industries, Ltd.), and then stained with hematoxylin-eosin (H&E) (commissioned to Applied Medical Research Laboratory) and observed with a microscope.

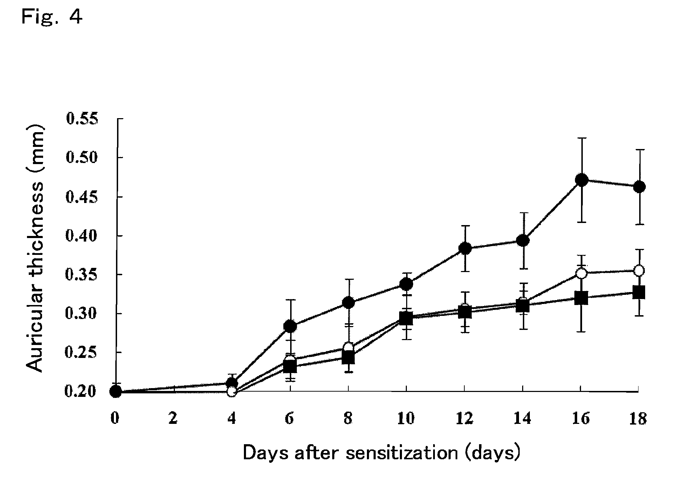

Fig. 4 is a graph showing a change over time of average auricular thickness in mice receiving oral administration of the exopolysaccharide-producing bifidobacterium after the beginning of the sensitization (five mice per group). The vertical axis represents the auricular thickness (mm), and the horizontal axis represents days after the sensitization (days). Filled circles represent the control (PBS alone) group, open circles represent the positive control (prednisolone) group, and filled squares represent the group receiving administration of the exopolysaccharide-producing bifidobacterium.

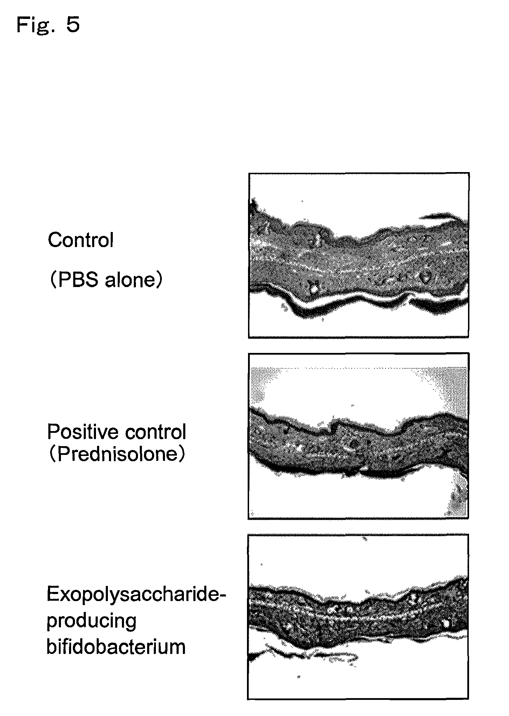

Fig. 5 shows photomicrographs of H&E-stained section from the auricles of treated mice including mice receiving oral administration of the exopolysaccharide-producing bifidobacterium on Day 20 after the beginning of the sensitization. Photomicrographs are, starting from the top, pictures of the control (PBS alone) group, the positive control (prednisolone) group and the group receiving administration of the exopolysaccharide-producing bifidobacteria.

Oral administration of viable cells of *Bifidobacterium longum* strain JBL05 (exopolysaccharide-producing bifidobacterium) significantly inhibited an increase of the auricular thickness as compared to the control group receiving administration of PBS alone (Fig. 4). The results of histological observation of the auricles also showed that oral administration of viable cells of *Bifidobacterium longum* strain JBL05 (exopolysaccharide-producing bifidobacterium) suppresses inflammation induced in the auricle and inhibits an increase of the auricular thickness (Fig. 5). Thus, viable *Bifidobacterium longum* strain JBL05 cells exhibited an anti-allergic effect.

### (Example 6: Anti-allergic effect by application of bifidobacterially produced polysaccharide)

In nine NC/Nga mice (7 week-old, male, Japan SLC, Inc.), 10 µl of 0.5 % 2,4,6-trinitro-1-chlorobenzene (TNCB) (Tokyo Chemical Industry Co., Ltd.) dissolved in acetone (Nacalai Tesque, Inc.) was applied to their right auricles (sensitization) and the same amount of acetone was applied alone to their left auricles, which were repeated twice every other day starting from Day 4 after the beginning of the TNCB application (sensitization). Mice were divided into three groups such that the auricular skin thickness was uniform (three mice per group). 20 µl of the purified polysaccharide fraction prepared in Example 1 dissolved in water (1 mg/ml) was applied to front and back of the auricle in each of three mice every day starting from Day 9 after the beginning of the sensitization. In a similar way, water as control was applied to another three mice and prednisolone in water (25 mg/ml) as positive control was applied to yet another three mice. TNCB was applied to the mice more than 30 minutes prior to application of the bifidobacterially produced polysaccharide, water or prednisolone every second day. The thickness of auricle was measured using a caliper for every TNCB application from the first day of the sensitization. The measurements were performed by the same examiner under the same conditions, and the average values were calculated (three mice per group).

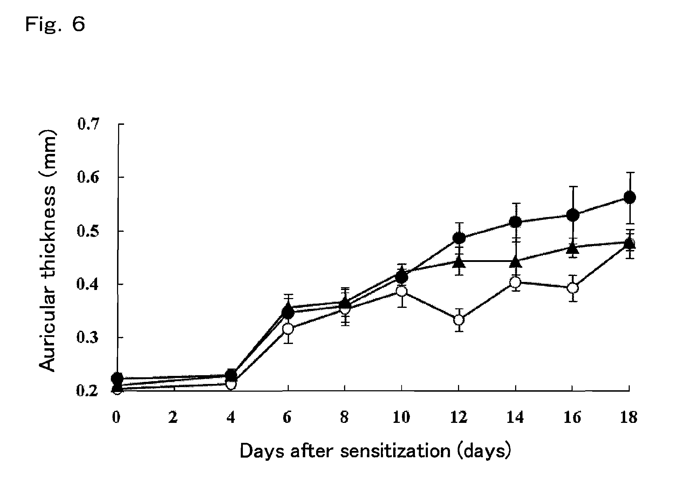

Fig. 6 is a graph showing a change over time of average auricular thickness in mice receiving application of the bifidobacterially produced polysaccharide after the beginning of the sensitization (three mice per group). The vertical axis represents the auricular thickness (mm), and the horizontal axis represents days after the sensitization (days). Filled circles represent the control (water alone)-applied group, open circles represent the positive control (prednisolone)-applied group and filled triangles represent the group receiving application of bifidobacterially produced polysaccharide.

Application of the purified polysaccharide fraction (bifidobacterially produced polysaccharide) inhibited an increase of the auricular thickness, as was the case with prednisolone (positive control), compared to the group receiving application of water only (control) (Fig. 6). Thus, the bifidobacterially produced polysaccharide also exhibited the anti-allergic effect via application.

### Industrial Applicability

The present invention makes it possible to provide an anti-allergic effect using a bifidobacterium that can dominantly inhabit the human intestine. The bifidobacterially produced polysaccharide or the exopolysaccharide-producing bifidobacterium can be formulated into food products, cosmetics, pharmaceutical products and the like to take advantage of anti-allergic effect thereof.

## Claims

1. An anti-allergic agent comprising a polysaccharide comprising galactose, glucose and rhamnose as constituents.

2. The anti-allergic agent according to claim 1, wherein the polysaccharide comprises galactose, glucose and rhamnose in a molar ratio of 3-5:1-3:1.

3. The anti-allergic agent according to claim 2, wherein the polysaccharide comprises the following structure.

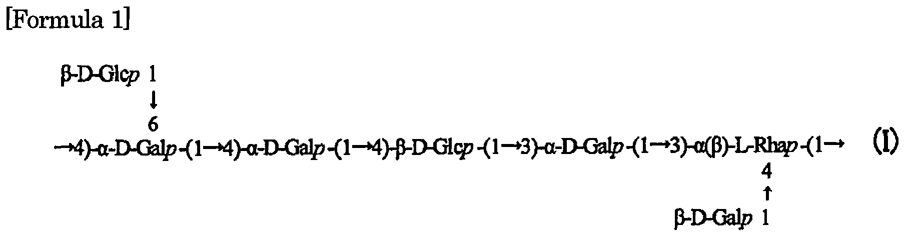

4. The anti-allergic agent according to any of claims 1 to 3, wherein the polysaccharide is obtained from a microorganism belonging to the genus *Bifidobacterium.*

5. The anti-allergic agent according to claim 4, wherein the microorganism belonging to the genus *Bifidobacterium* is *Bifidobacterium longum.*

6. The anti-allergic agent according to claim 5, wherein the *Bifidobacterium longum* is *Bifidobacterium longum* strain JBL05 (NITE BP-82).

7. An anti-allergic agent comprising a microorganism,
the microorganism belonging to the genus *Bifidobacterium* and producing an extracellular polysaccharide comprising galactose, glucose and rhamnose as constituents.

8. The anti-allergic agent according to claim 7, wherein the polysaccharide comprises galactose, glucose and rhamnose in a molar ratio of 3-5:1-3:1.

9. The anti-allergic agent according to claim 8, wherein the polysaccharide comprises the following structure.

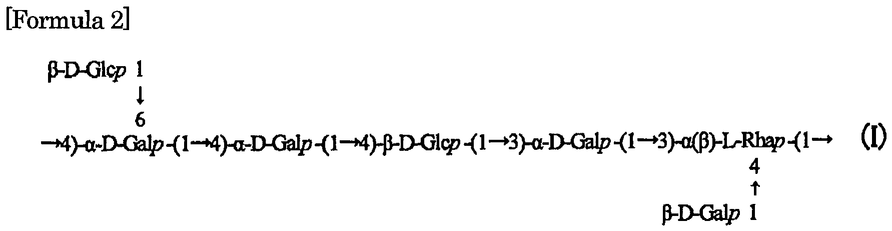

10. The anti-allergic agent according to any of claims 7 to 9, wherein the microorganism is *Bifidobacterium longum* strain JBL05 (NITE BP-82).

11. An oral composition comprising the anti-allergic agent of any of claims 1 to 10.

12. The oral composition according to claim 11, for suppressing atopic dermatitis or contact dermatitis.

13. A composition for external application comprising the anti-allergic agent of any of claims 1 to 6.

14. The composition for external application according to claim 13, for suppressing atopic dermatitis or contact dermatitis.
